# EUROPEAN PATENT SPECIFICATION

(11) **EP 0 442 579 B1**
(45) Date of publication and mention of the grant of the patent: **20.07.1994**
(21) Application number: 91200292.0
(22) Date of filing: 12.02.1991
(51) Int. Cl.: A61M 1/00, A61M 25/00

(54) **Drainage catheter**
Drainagekatheter
Cathéter de drainage

(30) Priority: 14.02.1990 NL 9000356
(43) Date of publication of application: 21.08.1991
(73) Proprietor: CORDIS EUROPA N.V., NL-9301 LJ Roden (NL)
(72) Inventor: Griep, Wilhelmus Antonius Maria, NL-9301 PK Roden (NL); Nap, Cornelis Philippus, NL-9354 AG Zevenhuizen (NL)
(74) Representative: 't Jong, Bastiaan Jacobus

(56) References cited:
- EP-A- 0 175 096
- US-A- 4 715 848
- US-A- 4 735 620
- US-A- 4 861 333

## Description

The invention relates to a drainage catheter according to the introductory part of claim 1.

Such a drainage catheter is known from US-A 4 715 848. This known catheter comprises a head piece in which a chamber is formed, in a side wall whereof the inlet opening is formed. A discharge tube is connected to the chamber through a restriction. A separate pressure tube is connected to a part of the pressure channel in the head piece, which ends in the nozzle opposite the restriction. In use a suction source is connected to the discharge tube, so that fluid together with debris can be drawn through the inlet opening, via the chamber in the discharge tube. Water can be supplied through the pressure tube. The water from the nozzle breaks up the debris drawn into the chamber through the inlet opening, so that debris parts can be taken up in the flow generated by the suction source. At the restriction or port between the chamber and the discharge tube a Venturi effect is generated, serving to further draw material into the chamber.

The invention has for its object to provide a drainage catheter of the type described in the preamble, which is simpler to use.

A drainage catheter according to the invention as characterized in claim 1, can be made with a very small diameter, so that it can be used for removing deposits and such from body vessels of a human being. Despite the small diameter of the catheter and accordingly of the discharge channel, a very good suction at the inlet opening is created due to the ejector action so that a source of liquid under pressure suffices for the operation of the catheter. During use only the feed of the liquid under pressure needs to be controlled. The suction generated is directly dependent on this feed. With the catheter according to the invention liquids and soft deposits and solid particles having a maximum size such that they can pass through the inlet opening, can be removed.

A further development of the catheter according to the invention is characterized in claim 2. During use a small quantity of the liquid under pressure will arrive in the vicinity of the front end of the catheter via the narrow passage openings. When a contrast fluid is used as liquid the vicinity of the front end of the catheter can in this way be made visible on an X-ray screen.

The step of claim 3 is preferably applied thereby. A narrow passage opening in the face of the front end of the catheter can cause contrast liquid to flow out in the forward line of the catheter whereby guiding of the catheter is simple while it is made visible on an X-ray screen.

A preferable embodiment is characterized in claim 4. The cross-section of the pressure channel in the portion bending back is in this way very carefully controlled and dimensioned, such that the flow of the liquid under pressure out of the pressure channel and along the inlet opening can create the desired ejector action with great reliability.

A further preferred development is characterized in claim 5. Even with catheters having a very small diameter a reliable ejector action can be obtained in this way.

The invention is further elucidated in the following description with reference to the annexed figures of an embodiment.
Fig. 1 shows schematically an embodiment of a drainage catheter according to the invention in the position of use;
fig. 2-5 show partly broken away perspective views of the front end of an embodiment of the catheter at different stages of manufacture;
fig. 6 shows the front end of the catheter in partly broken away perspective view in the position of use.
Fig. 7 shows the front end of another embodiment of the catheter according to the invention, in a view corresponding to that of fig. 6.
Fig. 8 shows a cross-section according to line VIII-VIII in fig. 7.

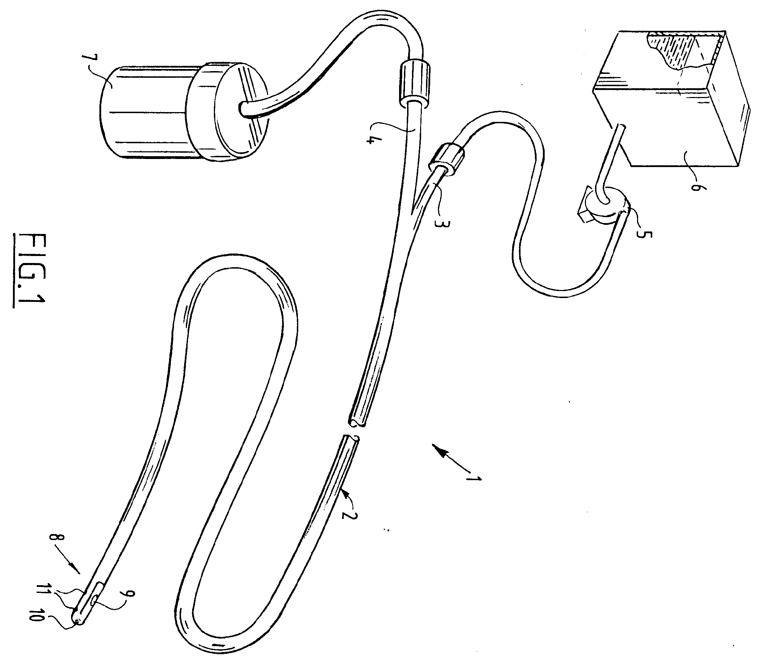

The drainage catheter 1 shown in fig. 1 comprises a tube-like basic body 2 with mutually separated pressure channel 3 and discharge channel 4.

Connected to the pressure channel 3 in the situation of use is a source of liquid under pressure, which is indicated in fig. 1 by a pump 5 which feeds liquid from a reservoir 6.

The discharge channel 4 is connected to a discharge reservoir 7 wherein substances discharged with the catheter are stored.

Formed in a side wall at the front end 8 of the catheter 1 is an inlet opening 9 whereby material can be drawn in in a manner to be described later. The front end 8 is also provided with narrow leakage openings 10, 11 for contrast liquid, which will also be further described later.

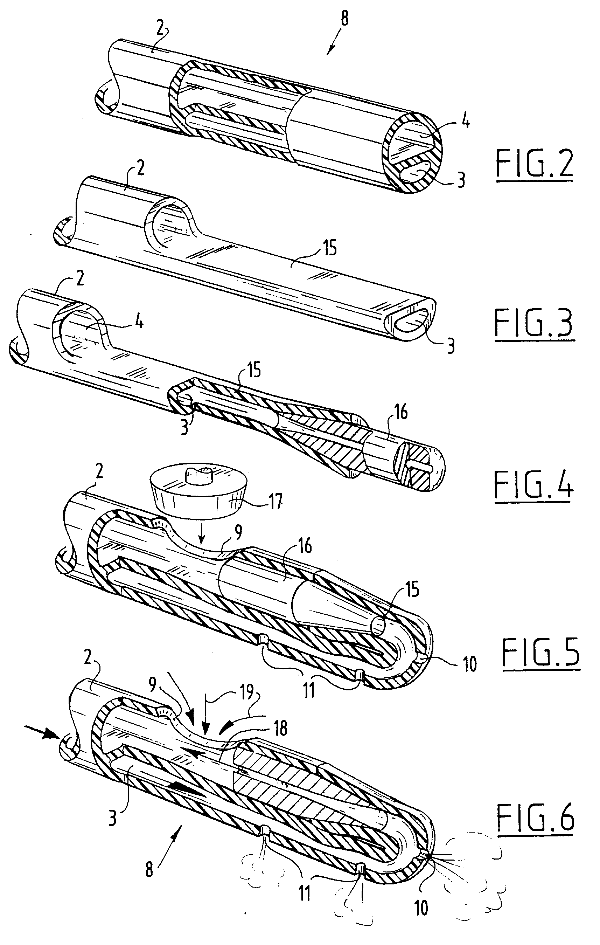

The figures 2-5 show schematically the manner in which the front end of the present embodiment can be manufactured.

The basic material is shown in fig. 2. As previously described this comprises a tube-like basic body 2 having therein a pressure channel 3 and a discharge channel 4. The pressure channel 3 has a smaller diameter than the discharge channel 4. The basic material can for example be manufactured by extrusion. From a long piece of extruded basic material a suitable length is removed and at the rear end connecting means for the pressure channel and discharge channel 3 and 4 respectively are arranged in the usual manner.

The outer wall portion which bounds the discharge channel 4 is ground away from the front end 8 of the basic material so that a tube-like pressure channel end 15 remains as shown in fig. 3. In this pressure channel end is fixed a jet nozzle 16, for example by glueing or ultrasonic welding.

The pressure channel end 15 is subsequently bent back and the nozzle 16 inserted into the front end of the discharge channel 4. This situation is likewise fixed by for example glueing or ultrasonic welding. The various material transitions are finished smoothly in the usual manner by grinding.

As is shown in fig. 5, an inlet opening 9 is then formed in the side wall of the basic body 2, for example by cutting it therein using a cutter 17.

During use of the catheter a liquid jet is directed along the inlet opening 9, as indicated in fig. 6 with the arrow 18 inside the discharge channel 4. Through the ejector action occurring therein there results a suction at the opening 9 whereby liquids, blood clots and small solid particles can be sucked up. This suction is schematically indicated with the arrows 19.

Close to the front end 8 a number of narrow leakage openings 10, 11 are arranged on the side remote from the inlet opening 9 in the outer wall of the basic body bounding the pressure channel 3. These leakage openings allow a small quantity of the liquid fed via the pressure channel 3 to escape. During use of the catheter a contrast fluid is used as liquid so that the liquid escaping via the openings 10, 11 makes the vicinity of the front end of the catheter visible on an X-ray screen. The leakage opening 10 in the leading end face of the catheter delivers leakage liquid in the forward line of the catheter so that the area towards which the catheter is moving is made properly visible on an X-ray screen.

The suction working is dependent on the speed and delivery of the liquid jet 18. When there is danger of the discharge channel becoming blocked a back-pressure occurs in the discharge channel at the position of the inlet opening 9 which immediately reduces the suction action. A self-regulating effect of the suction action is hereby automatically achieved.

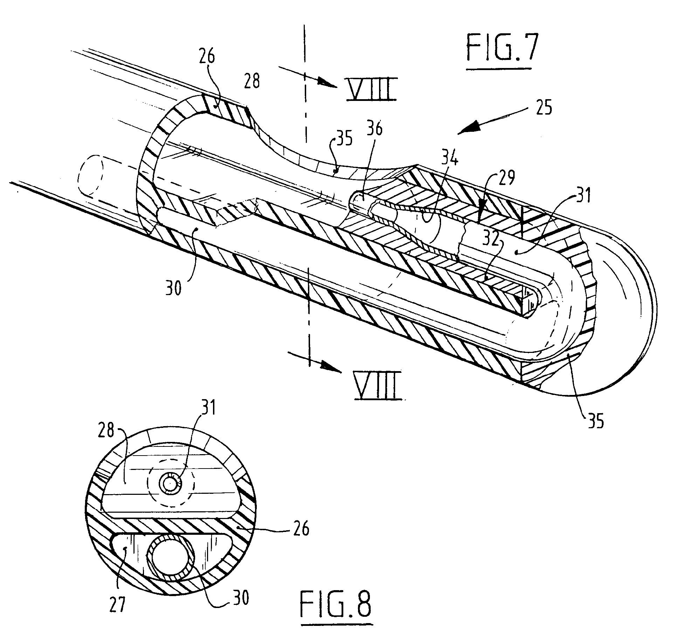

The catheter 25 as shown in fig. 7 also comprises a basic body 26 defining a pressure channel 27 and a discharge channel 28. A U-shaped metal tube 29 is arranged with its legs 30, 31 in respectively the pressure channel 27 and discharge channel 28. The U-tube is fixed in its place by hardened filling material 32. The outer end of the catheter is finished by applying a nose portion 33 of soft plastics material. The end portion is in the usual manner ground to a smooth surface.

The leg 31 of the U-shaped metal tube extending in the discharge channel is provided with a narrowed, tapered portion 34, so that a jet nozzle is formed at the end of the leg 31. This jet nozzle 36 directs the fluid which is supplied through the pressure channel in a accurately controlled manner along the inside of the inlet opening 35, so that the desired ejector action is obtained in a reliable and efficient way.

Instead of introducing contrast fluid by means of separate openings as the openings 10, 11 in figs. 5 and 6, the discharge of contrast fluid at the end of the catheter can also be controlled by controlling the outflow out of the discharge channel. If the discharge channel is blocked, the flow of fluid supplied through the pressure channel will have to escape through the inlet opening outwardly.

## Claims

1. Drainage catheter (1), comprising a mutually separated pressure channel (3) and a discharge channel (4), formed in one tube like basic body (2), connecting means at a rear end for connecting said pressure channel (3) and said discharge channel (4) to respectively a source (5) of liquid under pressure and discharge means (7) and wherein at the oppositely located front end said discharge channel (4) is connected with an inlet opening (9), formed in a side wall of the catheter (1), said pressure channel (3) bending back at the front end in the direction towards the rear end and forming a jet nozzle (16, 36), arranged in said front end of said discharge channel (4), characterized in that the discharge channel (4) has a continuous cross section from said jet nozzle (16, 36) on towards the rear end, the inlet opening (9) is formed in the side wall of the basic body bounding the discharge channel (4), the jet nozzle (16, 36) is capable of directing a liquid jet along said inlet opening (9) in such a way that at said inlet opening (9) of the catheter sufficient suction is created due to ejector action, without additional suction in the discharge means (7).

2. Catheter (1) as claimed in claim 1, wherein in the front end (8) at least one narrow passage opening (10, 11) is formed on a side remote from the inlet opening (9) in an outer wall portion of the basic body (2) bounding the pressure channel (3).

3. Catheter (1) as claimed in claim 2, wherein a narrow passage opening (10) is formed in the face of the front end (8) of the catheter (1).

4. Drainage catheter as claimed in claim 1 wherein the back bending part of the pressure channel is formed by a metal U-shaped tube (29) sealingly engaging with one leg (30) in said pressure channel and with a second leg (31) in said discharge channel (28).

5. Drainage catheter (25) as claimed in claim 4, wherein said second leg (31) of said U-shaped tube (29) is narrowed (34) toward its free end so as to form the jet nozzle (36).

## Patentansprüche

1. Drainagekatheter (1) mit zueinander getrenntem Druckkanal 3 und Ableitkanal 4, die in einem rohrförmigen Hauptteil (2) ausgebildet sind, Verbindungselementen an einem hinteren Ende, zum Verbinden des Druckkanals (3) und des Ableitkanals (4) mit jeweils einer unter Druck stehenden Flüssigkeitsquelle (5) und einer Ableiteinrichtung (7), und wobei der Ableitkanal (4) an dem gegenüberliegenden vorderen Ende mit einer Einlaßöffnung (9) verbunden ist, die in einer Seitenwand des Katheters (1) ausgebildet ist, wobei der Druckkanal (3) an dem vorderen Ende in die Richtung zum hinteren Ende umgebogen ist und eine Strahldüse (16, 36) bildet, welche in dem vorderen Ende des Ableitkanals (4) angeordnet ist, dadurch gekennzeichnet, daß der Ableitkanal (4) von der Strahldüse (16, 36) bis zum hinteren Ende einen kontinuierlichen Querschnitt aufweist, daß die Einlaßöffnung (9) in der Seitenwand des Hauptkörpers ausgebildet ist, welcher den Ableitkanal (4) eingrenzt, und daß die Strahldüse (16, 36) einen Flüssigkeitsstrahl entlang der Einlaßöffnung (9) lenken kann, so daß an der Einlaßöffnung (9) des Katheters ein ausreichender Sog aufgrund der Saugstrahlwirkung, ohne einen zusätzlichen Sog in der Ableiteinrichtung (7) erzeugt wird.

2. Katheter nach Anspruch 1, wobei in dem vorderen Ende (8) mindestens eine schmale Durchgangsöffnung (10, 11) auf einer von der Einlaßöffnung (9) entfernten Seite in einem Außenwandbereich des Hauptteils (2) ausgebildet ist, welcher den Druckkanal (3) eingrenzt.

3. Katheter nach Anspruch 2, wobei eine schmale Durchgangsöffnung (10) an der Vorderseite des vorderen Endes (8) des Katheters (1) ausgebildet ist.

4. Drainagekatheter nach Anspruch 1, wobei der umgebogene Teil des Druckkanals aus einem metallischen U-förmigen Rohr (29) gebildet ist, welches mit einem Schenkel (30) in den Druckkanal und mit dem zweiten Schenkel (31) in den Ableitkanal (28) dichtend eingreift.

5. Drainagekatheter (25) nach Anspruch 4, wobei der zweite Schenkel (31) des U-förmigen Rohres (29) in Richtung dessen freien Endes verjüngt ist, so daß die Strahldüse (36) gebildet wird.

## Revendications

1. Cathéter de drainage (1), comprenant un canal de pression (3) et un canal d'évacuation (4), mutuellement isolés, formés dans un corps de base d'aspect tubulaire (2), des moyens de raccordement à l'extrémité arrière pour relier respectivement ledit canal de pression (3) et ledit canal de décharge (4) à une source (5) de liquide sous pression et à un moyen d'évacuation (7), et dans lequel, à l'extrémité avant située à l'opposé, ledit canal d'évacuation (4) communique avec un orifice d'entrée (9), formé dans une paroi latérale du cathéter (1), ledit canal de pression (3) se retournant à l'extrémité avant dans la direction orientée vers l'extrémité arrière et formant une tuyère d'injection (16, 36), aménagée dans ladite extrémité avant dudit canal d'évacuation (4), caractérisé en ce que le canal d'évacuation (4) a une section uniforme depuis ladite tuyère d'injection (16, 36) en direction de l'extrémité arrière, l'orifice d'entrée (9) est formé dans la paroi latérale du corps de base jouxtant le canal d'évacuation (4), la tuyère d'injection (16, 36) est capable de diriger un jet liquide le long dudit orifice d'entrée (9) de telle façon qu'une dépression suffisante est créée audit orifice d'entrée (9) du cathéter, due à l'action d'éjection, sans aspiration complémentaire dans le moyen d'évacuation (7).

2. Cathéter (1) selon la revendication 1, dans lequel au moins une ouverture de passage restreint (10, 11) est formée dans l'extrémité avant (8) sur un côté, à distance de l'orifice d'entrée (9) dans une partie de paroi extérieure du corps de base (2) jouxtant le canal de pression (3).

3. Cathéter (1) selon la revendication 2, dans lequel une ouverture de passage restreint (10) est formée dans la surface de l'extrémité avant (8) du cathéter (1).

4. Cathéter de drainage selon la revendication 1, dans lequel la partie recourbée vers l'arrière du canal de pression est formée par un tube métallique (29) en forme de U dont un bras (30) est engagé de manière étanche dans ledit canal de pression et l'autre bras (31) est engagé dans ledit canal d'évacuation (28).

5. Cathéter de drainage (25) selon la revendication 4, dans lequel ledit second bras (31) dudit tube (29) en U est étranglé (34) vers son extrémité libre de manière à former la tuyère d'injection (36).
